Europäisches Patentamt

European Patent Office

Office européen des brevets

(19)

(11) Publication number: 0 275 834
A1

(12) **EUROPEAN PATENT APPLICATION**

(21) Application number: 87810776.2

(22) Date of filing: 22.12.87

(51) Int. Cl.⁴: **A61K 9/20** , A61J 3/10

(30) Priority: 29.12.86 US 947005

(43) Date of publication of application:
27.07.88 Bulletin 88/30

(84) Designated Contracting States:
DE ES FR GB GR IT NL SE

(71) Applicant: **WARNER-LAMBERT COMPANY**
**201 Tabor Road**
**Morris Plains New Jersey 07950(US)**

(72) Inventor: **Fisher, Christopher**
**46 Center Grove Rd. Apt. 501-U**
**Randolph New Jersey 07869(US)**
Inventor: **Smith, Kathleen Anne**
**2467 Rt 10 Abt 37-1A**
**Morris Plains New Jersey 07950(US)**
Inventor: **Brochu, David Paul**
**RD4 Box 4235-T**
**Stroudsburg, PA 18360(US)**
Inventor: **Martins, Eulalio Prudencio**
**66 Berlant Ave.**
**Linden New Jersey 07036(US)**

(74) Representative: **Silbiger, Jakob, Dr.**
**c/o CAPSUGEL AG Münchensteinerstrasse**
**41**
**CH-4002 Basel(CH)**

(54) Continuous process for producing a comestible tablet.

(57) A continuous process for producing a comestible tablet is disclosed. The process comprises continuously contacting different ingredients together used to prepare the comestible tablet under high shear conditions while atomizing a solvent for at least one of the ingredients into the mixing ingredients. The ingredients are mixed for a time sufficient to allow the ingredients to combine with each other in a substantially uniform manner to produce free flowing particles. The free flowing particles so produced have a substantially uniform distribution of ingredients from particle to particle. The particles are then dried and extruded or compacted into a comestible tablet.

# CONTINUOUS PROCESS FOR PRODUCING A COMESTIBLE TABLET

This invention relates to a continuous process for producing a comestible tablet. More particularly, this invention relates to a continuous process for producing an antacid tablet.

Many consumer products which are edible or ingestible are available in tablet form. Generally, these tablets, such as antacid tablets, contain multiple ingredients and are produced by batch step processes. Sometimes, because of the physical characteristics of the ingredients being blended together, there is difficulty in obtaining uniform blending of the ingredients. Thus, the amount of ingredients may vary from tablet to tablet as well as varying in distribution throughout the tablet.

A significant benefit to the art would be a continuous process for producing a comestible tablet in which there is a greater ability to control density and particle size resulting from mixed and combined ingredients, in which there is better control over the mixing of ingredients, and in which there is better mixing of ingredients resulting in a more uniform distribution of combined ingredients from particle to particle and hence from tablet to tablet. Such a benefit is provided by this invention.

This invention provides a continuous process for producing a comestible tablet comprising multiple ingredients. In preparing the tablet at least two different ingredients are combined with each other to form a free flowing particle. The ingredients are combined with each other continuously by high intensity mixing or mixing under high shear conditions. During mixing a solvent for at least one of the ingredients is atomized into the ingredients being mixed. The ingredients are mixed together for a time sufficient to allow the combination of ingredients into the desired particle size. The particles so produced have a substantially uniform distribution of the combined ingredients and the particles so produced are free flowing.

These particles may, upon drying, be extruded or compacted to produce tablets. If desired other ingredients may be added with the particles to provide the desired tablet.

This invention also provides a continuous process for producing consumable free flowing particles comprising substantially uniformly distributed ingredients. The process comprises mixing the ingredients together using high intensity mixing or under conditions of high shear. During such mixing a solvent for at least one of the ingredients is atomized into the ingredients being mixed thereby producing particles which are free flowing and substantially uniform in the distribution of ingredients from particle to particle.

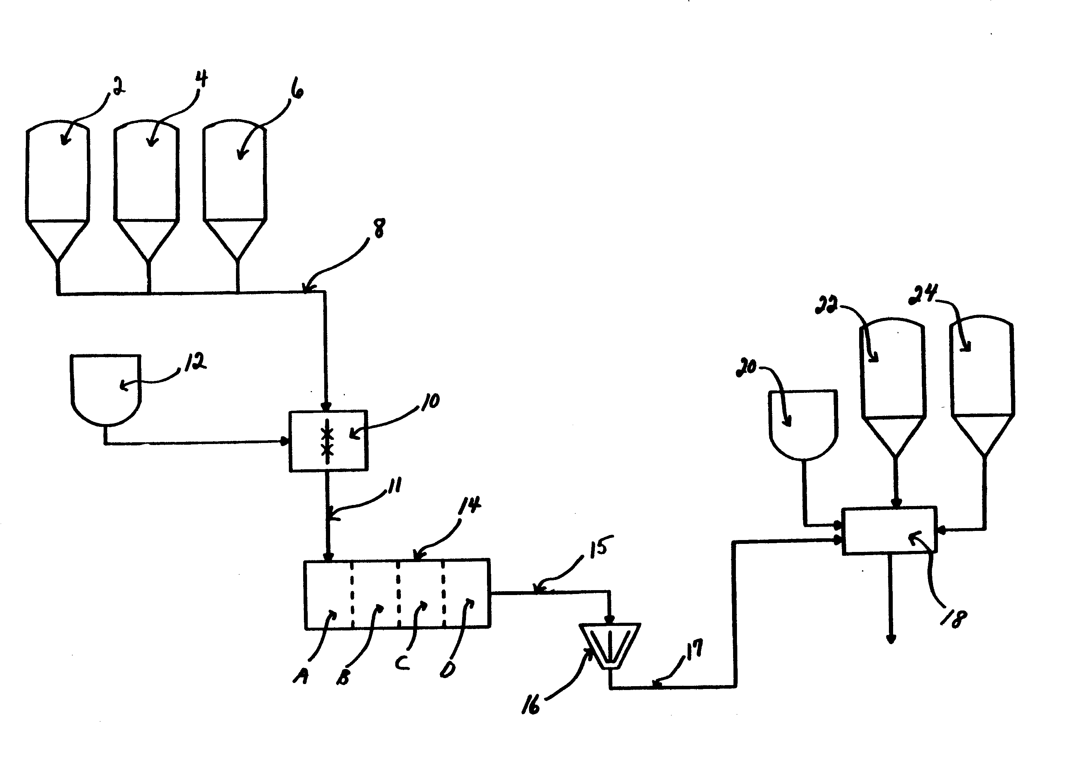

The drawing is a schematic flow sheet of a process of this invention.

This invention provides a continuous process for producing a comestible tablet, which comprises: continuously contacting different ingredients together used to prepare the comestible tablet under high shear conditions while atomizing a solvent for at least one of said ingredients; mixing the ingredients for a time sufficient to allow the ingredients to combine with each other in a substantially uniform manner to produce free flowing particles having a substantially uniform distribution of ingredients from particle to particle; drying said free flowing particles; and extruding or compacting the particles into a comestible tablet.

This invention also provides a process for producing an antacid tablet which comprises: continuously contacting at least one sweetener and an antacid together under high shear conditions while atomizing water or a solution comprising water and up to about 85 wt % of said sweetener into the mixing sweetener and antacid; mixing the sweetener and antacid for a time sufficient to allow the sweetener and antacid to combine with each other in a substantially uniform manner, to produce free flowing particles having a substantially uniform distribution of sweetener and of antacid from particle to particle; drying said free flowing particles; and extruding or compacting said particles into an antacid tablet.

Any ingredients which are capable of being combined into the same particle may be combined by the process of this invention. The particles so produced will have a substantially uniform distribution of ingredients from particle to particle. Without wishing to be limited by any particular definition or theory, by "combined" it is meant that the ingredients are agglomerated, aggregated, granulated, or the like, by which means one or more ingredients are absorbed onto or into, entrapped within, coated with, surrounded by, embedded in, dissolved in, or the like, into another ingredient to form a particle comprising the different ingredients.

The process of this invention is particularly useful where the ingredients being combined are of different relative particle sizes. In such cases, this invention overcomes the problems of obtaining a uniform distribution of the relatively smaller sized particle ingredient within the relatively larger sized particle ingredient. For example, in the production of antacid tablets it is highly desirous to have a uniform distribution of the antacid ingredient throughout the tablet. Since the antacid component is usually blended with a sweetener it is sometimes difficult to obtain a uniform distribution of the smaller particle sized antacid ingredient with the larger particle sized sweetener throughout the tablet. This invention provides a process to obtain just such a uniform distribution.

The sweeteners used in the practice of this invention may be selected from a wide range of materials, including water-soluble sweetening agents, water-soluble artificial sweetening agents, and dipeptide based sweeteners, including mixtures thereof. Without being limited to particular sweeteners, representative illustrations encompass:

A. Water-soluble sweetening agents such as monosaccharides, disaccharides, and polysaccharides such as xylose, ribose, glucose (dextrose), mannose, galactose, fructose (levulose), sucrose, maltose, invert sugar (a mixture of fructose and glucose derived from sucrose), lactose, cellubiose, partially hydrolyzed starch, corn syrup solids and sugar alcohols such as sorbitol, xylitol, mannitol and mixtures thereof;

B. Water-soluble artificial sweeteners such as the soluble saccharin salts, i.e., sodium or calcium saccharin salts, cyclamate salts, acesulfam-K and the like, and the free acid form of saccharin; or

C. Dipeptide based sweeteners such as L-aspartyl-L-phenylalanine methyl ester and materials described in U.S. Patent No. 3,492,131 and the like.

In general, the amount of sweetener is primarily a matter of taste preference and will vary with the sweetener selected and with the ingredients in the comestible tablet being prepared. The water-soluble sweeteners described in category A above are preferably used in amounts of about 25% to about 75% by weight, and most preferably from about 40% to about 70% by weight, based on the weight of the total comestible tablet. Generally speaking a disaccharide, particularly sucrose, is preferred since it also provides bulk and texture to the comestible tablet.

In those instances where the sweetener chosen does not provide bulk, such as where artificial sweeteners are used, the term sweetener, for purposes of this invention, is meant to include artificial sweeteners and bulking agents. Typical bulking agents such as maltodextrin, polydextrose, and the like, are utilized in amounts of about 25% to about 72.5% by weight, together with one or more of the artificial sweeteners described in Categories B and C above, which artificial sweeteners are utilized in amounts of about 0.005% to about 5.0%, most preferably about 0.05% to about 2.5% by weight, based on the weight of the total comestible tablet. The artificial sweeteners and the bulking agents generally provide approximately equivalent levels of bulk and sweetness as do the sweeteners, particularly the disaccharides, from category A above. Thus, about 25% to about 75%, preferably about 40% to about 70% by weight of one of more sweeteners, based on the weight of the total comestible tablet, are used in the practice of this invention. The amounts of sweetening agents described above are ordinarily necessary to achieve a desired level of sweetness independent of the flavor level achieved from the inclusion of flavoring agents.

The sweetener can be granulated or pulverized and usually comprises mean particle sizes within the range of about 100 to about 500 microns with about 150 to about 450 being preferred. A pulverized sweetener such as sucrose will generally have a mean particle size within the range of about 140 to about 340 microns with about 240 microns being preferred. A granulated sweetener such as sucrose will generally have a mean particle size within the range of about 240 to about 440 microns with about 340 microns being preferred. Generally, the sweetener has a maximum moisture content of not more than about 6 wt %. Preferably the moisture content is within the range of about 2 to about 4 wt %.

Antacids which may be used include those well known to those skilled in the art. Examples include sodium carbonate, sodium bicarbonate, calcium carbonate, magnesium carbonate, a codried gel of aluminum hydroxide and magnesium carbonate, aluminum hydroxide, magnesium hydroxide, hydroxymagnesium aluminum sulfate, aluminum carbonate, dihydroxy aluminum sodium carbonate (DASC), aluminum magnesium hydroxide monohydrate (magaldrate), mixtures thereof, and the like.

The antacid usually comprises mean particle sizes within. the range of about 1 to about 100 microns with about 1 to about 50 microns being preferred. An antacid such as DASC normally has a mean particle size within the range of about 6 to about 36 microns with about 16 microns being preferred. The antacid ingredient generally has a moisture content of not more than about 15 wt %. Preferably, the moisture content is within the range of about 2 to about 9 wt %. The antacid can be used in amounts up to about 50 wt %, preferably about 20 to about 40 wt % and most preferably about 20 to about 35 wt %.

Without wishing to be bound by theory it is believed that the solvent utilized, which solvent may be water or other liquid or mixture of liquids utilizable in the production of comestible products, dissolves at least a portion of a particle of one of the ingredients thus allowing another (different) ingredient to become absorbed onto or into (or embedded in or entrapped in) the surface of the partially dissolved particle resulting in one final particle comprising at least two different ingredients. Thus, for example, the particle which is at least partially dissolved may be viewed as a carrier for the other ingredient particles. As used herein "solvent" also includes a solution comprising the solvent and at least one of the ingredients. Alternatively, and once again, without wishing to be bound by theory, it is believed that the solution comprising at least one of the ingredients may act, in addition to at least partially dissolving a portion of one of the ingredient particles, as a binder for the different ingredients being mixed together. In the production of antacid tablets for example, the preferred solvent is water for the preferred sweetener, which is sucrose, or is a solution of up to about 85% by weight sucrose. When the water or the sucrose solution is atomized, and contacted with another particle such as an antacid particle, the antacid ingredient combines with the sucrose to produce a free flowing particle containing both ingredients.

The ingredients are mixed together under conditions of high shear or high intensity mixing while the aforedescribed solvent or solution is atomized into the mixture. This mixing takes place in any convenient device capable of providing the necessary mixing conditions. The residence time in the mixing device is sufficient to allow the ingredients to combine to form free flowing particles of the desired size, but not so long as to form too large a particle size which will not be free flowing. Generally, the mixing of the ingredients and the formation of the free flowing particles occurs almost simultaneously. By "free flowing" it is meant that the particles are easily removable from the mixing device without clogging the device and that they have a reduced resistance to flow. Once the particles having the combined ingredients are formed they are dried and then extruded or compressed or compacted into a tablet using conventional tableting equipment.

Other ingredients normally utilized in the tableting art may be utilized to produce the comestible tablets in accordance with the practice of this invention. Such ingredients may include binders, lubricants, glidants, anti-adherents, disintegrants, colors, flavors, and the like. The ingredients generally known as disintegrants may be blended or mixed with the sweetener and antacid to produce the free flowing particles. Otherwise, these additional ingredients are normally added to the free flowing particles to form a tableting mixture. The tableting mixture is blended into a homogeneous mixture which is then extruded or compacted or compressed into a tablet using conventional tableting equipment.

Examples of binders which may prove useful are acacia, tragacanth, gelatin, starch, cellulose materials such as methylcellulose and sodium carboxymethylcellulose, alginic acid and salts of alginic acid, magnesium aluminum silicate, polyethylene glycol, guar gum, polysaccharide acids, bentonites, and the like. The binders may be used in amounts up to about 50 wt % and preferably about 10 to about 40 wt %.

A "lubricant" as used herein means agents that act between surfaces in relative motion to prevent friction and wear. In the manufacture of comestible tablets, lubricants may be used, for example, to prevent excess wear on dies and punches. True lubricant action is particularly required immediately after compression of the tablet within the die to reduce the friction between the inner die wall and the tablet edge during the ejection cycle. The absence of a lubricant at this stage may cause straining of the press parts and tablets formed thereby may be damaged. The lubricants which may prove useful are among those well known in the art. Representative examples may be selected from the group consisting metallic stearates (e.g., magnesium stearate, calcium stearate, zinc stearate, and the like), hydrogenated vegetable oil, partially hydrogenated vegetable oils, animal fats (e.g. triglycerides), polyethylene glycols, polyoxyethylene monostearate, talc, light mineral oils, sodium benzoate, sodium lauryl sulfate, magnesium lauryl sulfate, and mixtures thereof. Of particular usefulness are magnesium stearate, calcium stearate and mixtures thereof. Hydrogenated cottonseed oil, solid vegetable oil, hydrogenated soya oil and mixtures thereof are the preferred oil lubricants. In general, the lubricant or mixture of lubricants is present in the tableting mixture in amounts less than about 5% by weight and preferably about 0.25 to about 2% by weight based on the comestible tablet. If oil lubricants are used, it is preferred they be present in amounts of about 0.4% to about 1% by weight of the comestible tablet.

To aid in the free flow of the particles or tableting mixture in the tableting equipment, a glidant may be used. Glidants reduce interparticulate friction and assure smooth and uniform flow of materials from larger apertures to smaller apertures in the tableting equipment. Fluidity of the particles or tableting mixture is not only important as to the direct effect on uniformity of die fill, and thus uniform tablet weight, but also plays a role in proper homogeneity. The contribution a glidant makes toward improving fluidity depends on the chemical nature of the glidant relative to that of the particles or tableting mixture, i.e. on the presence of unsaturated valencies or bonds which have potential chemical interaction; and upon physical factors such as size and shape distribution of both the glidant particles and the particles or tableting mixture.

For any particular system there is usually an optimum concentration above which the glidant may start to act as an antiglidant. The optimum level depends on several factors, one of which is the moisture level of the particles or tableting mixture. The amount of glidant used may be from about 0.5% to about 5% by weight of the comestible tablet. Those glidants which may be useful are selected from the group consisting of alkali metal salts, talc, starch, polyhydric alcohols and mixtures thereof. Preferably the glidant is selected from the group consisting of calcium carbonate, mannitol, sorbitol and mixtures thereof present in amounts of about 1% to about 2.5% by weight of the comestible tablet.

Anti-adherents function to prevent the particles or tableting mixture from sticking to the faces of the punches and the die walls, but most importantly prevent adherence of the particles or tableting mixture granules to one other, a phenomenon known as blocking. Anti-adherents may be added to the particles or tableting mixture. The anti-adherent is selected from the group consisting of silicates, silicon dioxide, talc and mixtures thereof present in amounts of about 0.2% to about 1% by weight of the comestible tablet and preferably about 0.3 to about 0.6% by weight. Generally the anti-adherent is a finely divided, low bulk density powder which is preferably water insoluble. The preferred anti-adherents are fumed silica and talc. The term fumed silica is meant to include pyrogenic silicas, micron sized silicas and hydrated silicas.

It is apparent from the above discussion of the lubricants, glidants and anti-adherents that certain materials may be useful as more than one ingredient. However, frequently one material may function well in one mechanism, e.g., hydrogenated vegetable oil as a lubricant, but will be ineffective or detrimental toward another mechanism, e.g., hydrogenated vegetable oil may tend to actually retard the flow characteristics of certain solids, making it a poor glidant. Thus, when selecting the specific combination of these three ingredients, attention should be given to maximize the advantages of each ingredient and to prevent unnecessary cancellation of one ingredient's effectiveness. Such a determination is well within the capabilities of those skilled in the art without the need for undue experimentation.

Disintegrants may be used to facilitate the breakup of the comestible tablet during its intended use. Examples of disintegrants which may be used include starches such as corn starch, potato starch, and modified corn starch, and the like. The disintegrant may be used in amounts of up to about 10 wt % with about 4 to about 6 wt % being preferred. These disintegrants are preferably combined with the sweetener and antacid to produce the free flowing particles. Other disintegrants which may be useful include clays such as bentonite; microcrystalline cellulose; alginates; gums such as agar, guar, locust bean, karaya, pectin, and tragacanth; and the like.

The disintegrants usually comprise particle sizes within the range of about 10 to about 200 microns with about 10 to about 100 microns being preferred. An ingredient such as corn starch, normally used in comestible tablets, usually has a mean particle size within the range of about 10 to about 30 microns, with about 20 microns being preferred. Generally, the disintegrants have a maximum moisture content of not more than about 10 wt %. Preferably the moisture content is within the range of about 2 to about 8 wt %.

Coloring agents (colorants or colors) which may be used include titanium dioxide and may be used in amounts up to about 2 wt % and preferably up to about 1 wt %. Also, the colorants may include other dyes suitable for food, drug and cosmetic applications, and known as F.D. & C. dyes and lakes. The materials acceptable for the foregoing spectrum of use are preferably water-soluble, and include indigoid dye, known as F.D. & C. Blue No. 2, which is the disodium salt of 5,5-indigotindisulfonic acid. Similarly, the dye known as F.D. & C. Green No. 1 comprises a triphenylmethane dye and is the monosodium salt of 4-[4-N-ethyl-p-sulfobenzylamino)diphenylmethylene]-[1-N-ethyl-N-p-sulfoniumbenzyl)-Δ2,5-cyclohexadienimine]. A full recitation of all F.D. & C. and D. & C. dyes and their corresponding chemical structures may be found in the Kirk-Othmer Encyclopedia of Chemical Technology, at Volume 5, Pages 857-884, which text is accordingly incorporated herein by reference.

Flavoring agents (flavors) well known to those skilled in the art can be added. These flavoring agents may be chosen from synthetic flavor oils and flavoring aromatics, and/or oils, oleo resins and extracts derived from plants, leaves, flowers, fruits and so forth, and combinations thereof. These flavoring agents are generally liquids. However, they can also be used as spray dried solids. The use of flavoring agents having other distinct physical forms such as powdered flavorings, beaded flavorings and encapsulated flavorings may also be used. Representative flavor oils include: spearmint oil, cinnamon oil, oil of wintergreen (methylsalicylate), peppermint oils, clove oil, bay oil. anise oil, eucalyptus oil, thyme oil, cedar leaf oil, oil of nutmeg, oil of sage, oil of bitter almonds, and cassia oil. Also useful are artificial, natural or synthetic fruit flavors, such as vanilla, and citrus oil, including lemon, orange, grape, lime and grapefruit and fruit essences including apple, pear, peach, strawberry, raspberry, cherry, plum, pineapple, apricot and so forth. Flavoring agents such as aldehydes and esters including cinnamyl acetate, cinnamaldehyde, citral diethylacetal, dihydrocarvyl acetate, eugenyl formate, p-methylamisol, and so forth may also be used. Generally any flavoring or food additives such as those described in Chemicals Used in Food Processing, pub 1974 by the National Academy of Sciences, pages 49-53 and 63-258 may be used.

The amount of flavoring agent employed is normally a matter of preference subject to such factors as flavor type, tablet type and strength desired. In general, amounts of about 0.5% to about 3.0% by weight of the comestible tablet are useable with amounts of about 0.3% to about 1.5% being preferred and about 0.7% to about 1.2% being most preferred.

Fillers may also be used if desired in amounts up to about 50 wt % with about 4 to about 30 wt % being preferred. Such fillers may include aluminum hydroxide, alumina, aluminum silicates, calcium carbonate and mixtures thereof. Some of the fillers, as will be seen from the above description of antacids, may also function as an antacid. When the filler chosen may also function as an antacid, the amount of antacid and such filler used may be appropriately formulated without undue experimentation by those skilled in the art in order to provide the desired level of antacid activity.

Additional information relating to ingredients which may be utilized in producing the comestible tablets may be found in Banker, G.S., Peck, G.E. and Baley, G., "Tablet Formulation and Design." In: Lieberman, H.A., and Lachman, L. (Editors), Pharmaceutical Dosage Forms: Tablets, (Marcel Dekker, Inc., 1980), Volume 1, pages 61 to 107, the disclosure of which is incorporated herein by reference thereto.

The process of this invention will be better understood by reference to the drawing, the description for which is set forth hereinafter. While the description provided is directed to the continuous process for the production of an antacid tablet, it is to be understood that the process is not to be so limited. Other types of tablets and multi-ingredient particles may be produced by the appropriate substitution of ingredients necessary for obtaining the desired product. For example, breath freshening type tablets that may contain the combinations of ingredients described heretofore but instead of an antacid ingredient there will be a breath freshening ingredient. The breath freshening ingredients may be those solids and liquids well known to those skilled in the art. Examples include flavors; chlorophyll; metallic salts such as copper gluconate, zinc chloride, and the like; natural vegetable oils such as cottonseed oil; and the like.

In the drawing hoppers 2, 4 and 6 contain some or all of the ingredients to be combined with each other to produce free flowing particles comprising such ingredients. The order of the ingredients in the hoppers is not critical. Reference to the particle sizes of the ingredients utilized in the production of the antacid tablet exemplified hereinafter clearly demonstrates the combineability of ingredients of vastly different particle sizes. Thus, ingredients can be combined where at least one ingredient comprises particles that are about 15 to about 20 times smaller than the particle size of another ingredient.

Thus, hopper 2 may contain sweetener, hopper 4 may contain a disintegrant, and hopper 6 may contain an antacid. The ingredients of hoppers 2, 4 and 6 are deposited onto conveyor belt 8 which brings them to the high intensity mixer 10. In high intensity mixer 10 the ingredients are mixed under conditions of high shear while a solvent for at least one of the ingredients, or a solution comprising at least one of the ingredients, contained in storage tank 12, is continuously spray atomized into the mixing ingredients in high intensity mixer 10. Usually, the droplet size is not greater than a mean superficial diameter of 200 microns. Free flowing particles will be produced almost immediately and are passed through line 11 into continuous fluid bed dryer 14. The particles are dried in fluid bed drier 14 which has zones A, B, C and D and are then passed through line 15 into sizing device 16 from which they proceed through line 17 to homogeneous mixer 18. In homogeneous mixer 18 the particles may be combined with other ingredients commonly used in the tablet being produced to provide the tableting blend or mixture. The tableting blend is then extruded or compacted or compressed using conventional tableting equipment into the finished tablet. Thus, storage tank 20 may contain a liquid flavoring agent, hopper 22 may contain a powdered flavoring agent, and hopper 24 may contain a lubricant or other processing aid for tableting.

The ingredients in hoppers 2, 4 and 6 are generally maintained at ambient conditions, that is within the range of about 20 to about 30°C. The relative humidity is not critical and can range from about 20 to about 90%. A suitable set of temperature and humidity conditions can be, for example, a temperature of about 24°C to about 32°C and a relative humidity of about 40% to about 85%.

The high intensity mixer 10 is commercially available and is sometimes referred to as a continuous granulator. Generally, the ingredients on conveyor 8 are delivered into high intensity mixer 10 at any suitable rate sufficient to achieve maximum contact. The ingredients are mixed together by the action of rotators or impellers revolving at high speeds such as about 1000 to about 5000 RPM. A suitable rotating speed for blending the exemplified antacid tablet ingredients described heretofore is about 3500 RPM.

Storage tank 12 contains a solvent, such as water or a solution of sweetener such as sucrose. When a solution is used it can contain up to about 85 wt % sweetener with about 40 to about 70 wt % being preferred. The contents of storage tank 12 are preferably maintained at a temperature not greater than about 38°C (100°F) and may be about 20 to about 30°C. The solvent or solution in storage tank 12· is spray atomized into high speed mixer 10 concurrently with the addition of the ingredients from conveyor belt 8. Atomization of the solvent or solution is at a rate sufficient to allow the combination of ingredients into free flowing particles and may be about 23 to about 68 kg/hr. The solvent or solution is sufficiently atomized to provide the means whereby the ingredients combine to produce free flowing particles of the desired size having a substantially uniform distribution of ingredients from particle to particle.

The temperature maintained within high speed mixer 10 is not critical, however, it may conveniently be at a temperature within the range of about 0°C to about 50°C. The residence time of the ingredients in high speed mixer 10 is sufficiently long enough to allow the mixing ingredients to combine to form free flowing particles of the desired size which have the ingredients substantially uniformly distributed from particle to particle. Generally the residence time is not more than 1 second. Preferably the residence time is within the range of about 0.05 to about 1 second and most preferably about 0.5 seconds.

The particles produced in high intensity mixer 10 generally have a uniform mean particle size within the range of about 250 to about 600 microns, The ingredients exemplified heretofore for producing the antacid tablet generally produce particles having a maximum mean particle size of about 500 microns. The particles produced in high intensity mixer 10 usually have a maximum moisture content of not more than about 15 wt % with a preferred moisture content of about 5 to about 10 wt %.

The particles exiting high intensity mixer 10 enter continuous fluid bed dryer 14 at a continuous rate consistent with the input/output of the high intensity mixer 10. Normally, the particles entering the continuous fluid bed dryer 14 are at ambient temperature (24-30°C). The residence time of the particles in the continuous fluid bed dryer 14 is for a time sufficient to dry the particles without adversely effecting their desired characteristics. The drying time is normally about 5 minutes to about 1 hour. The particles are dried at a temperature sufficiently high enough to promote drying at a reasonable rate but not so high as to cause degradation of the particles or as to cause adverse effects upon the desired particle characteristics. For example, when sucrose is the sweetener, the drying temperatures should not be so high as to cause the sugar to reach its carmelization temperature. Typically, the particles being dried reach a temperature within the range of about 45 to about 60°C with a preferred maximum temperature being about 57°C (135°F). Generally, when drying is complete the particles are at a temperature within the range of about 28 to about 40°C with a preferred maximum of about 38°C (100°F). The blower inlet of continuous fluid bed dryer 14 usually provides air at a rate sufficient to fluidize the particles and to reduce the moisture content to within a range of about 0.1 to about 1 wt %.

Preferably continuous fluid bed dryer 14 contains several consecutive drying zones arranged so that the free flowing particles pass through the zones at a downward slope. As shown in the drawing continuous fluid bed dryer 14 may have four zones, Zone 1, 14A; Zone 2, 14B; Zone 3, 14C; and Zone 4, 14D. The inlet air temperatures of the zones can be as follows: Zone 1, about 35 to about 55°C with about 40 to about 50°C being preferred; Zone 2, about 50 to about 70°C with about 55 to about 65°C being preferred; Zone 3, about 70 to about 90°C with about 75 to about 85°C being preferred; and Zone 4, about 25 to about 40°C with about 30 to about 35°C being preferred. Suitable operating air inlet temperatures, for example, can be: Zone 1, about 45°C; Zone 2, about 60°C; Zone 3, about 80°C; and Zone 4, a maximum of about 32°C.

The dried particles exiting continuous fluid bed dryer 14 usually have a maximum moisture content of not more than 2 wt %. Preferably, the moisture content is within the range of about 0.1 to about 1 wt %. The dried particles exiting the continuous fluid bed dryer 14 are generally at a temperature within the range of about 35 to about 46°C and normally, the exiting dried particles will not be at a temperature higher than about 46°C.

The dried particles next proceed through line 15 into sizing device 16. Generally, the maximum temperature of the sized particles does not exceed about 43°C. The sized particles have a mean particle size within the range of about 250 to about 600 microns with a suitable mean particle size for the purposes of this illustration being about 450 microns. Sizing device 16 is commercially available and is sometimes referred to as a comminuting machine.

The sized particles next proceed through line 17 to homogeneous mixer 18 where they may be blended with other ingredients, if desired, to form the final tableting composition. Thus, for example, a liquid flavoring agent, such as oil of spearmint, may be contained in storage tank 20; a powdered flavoring agent, such as wintergreen (methyl salicylate) may be contained in hopper 22; and a lubricant or processing aid, such as magnesium stearate, may be contained in hopper 24. The desired ingredients are added to mixer 18 and mixed for a time sufficient to provide a homogeneous blend. Suitable mixing times range from about 1 to about 20 minutes with about 5 to about 10 minutes being preferred. Homogeneous mixer 18 may be what is commonly referred to as a ribbon blender and does not constitute a critical aspect of this invention.

The mixture exiting homogeneous mixer 18 is then passed to a conventional tableting machine, not shown, where it is extruded or compacted into the desired final tablet.

Those skilled in the art will appreciate that if just the free flowing particles are desired and not the finished tablet, the particles may be collected either after exiting continuous fluid bed dryer 14 or sizing device 16.

As used herein the terms "wt %" and "% by weight" have the same meaning and, unless indicated otherwise, are based on the weight of the comestible tablet.

The invention being thus described, it will be obvious that the same may be varied in many ways. Such variations are not to be regarded as a departure from the spirit and scope of the invention and all such modifications are intended to be included within the scope of the claims.

## Claims

1. A continuous process for producing a comestible tablet, which comprises: continuously contacting different ingredients together used to prepare the comestible tablet under high shear conditions while atomizing a solvent for at least one of said ingredients; mixing the ingredients for a time sufficient to allow the ingredients to combine with each other in a substantially uniform manner, to produce free flowing particles having a substantially uniform distribution of ingredients from particle to particle; drying said free flowing particles; and extruding or compacting the particles into a comestible tablet.

2. The process of Claim 1 wherein said ingredients have different particle sizes with at least one ingredient being a relatively large particle and at least one ingredient being a relatively small particle.

3. The process of Claim 1 wherein an ingredient is a sweetener.

4. The process of Claim 1 wherein an ingredient is an antacid.

5. The process of Claim 1 wherein the solvent is a solution comprising a sweetener.

6. The process of Claim 5 wherein the sweetener is selected from the group consisting of glucose, fructose, xylose, ribose, mannose, galactose, invert sugar, sucrose, maltose, lactose, cellobiose, partially hydrolyzed starch, corn syrup and mixtures thereof.

7. The process of Claim 1 wherein the antacid is selected from the group consisting of sodium carbonate, sodium bicarbonate, aluminum hydroxide, magnesium hydroxide, aluminum carbonate, dihydroxyaluminum sodium carbonate, aluminum magnesium hydroxide monohydrate, calcium carbonate, magnesium carbonate, a codried gel of aluminum and magnesium carbonate, and mixtures thereof.

8. The process of Claim 7 wherein the antacid is dihydroxyaluminum sodium carbonate and the sweetener is sucrose.

9. The process of Claim 1 wherein mixing of the ingredients and formation of the free flowing particles occurs almost simultaneously.

10. A continuous process for producing an antacid tablet, as claimed in anyone of the Claims 1 to 9, which comprises: continuously contacting at least one sweetener and an antacid together under high shear conditions while atomizing water, or a solution comprising water and up to about 85 wt% of said sweetener, into the mixing sweetener and antacid; mixing the sweetener and antacid for a time sufficient to allow the sweetener and antacid to combine with each other in a substantially uniform manner to produce free flowing particles having a substantially uniform distribution of sweetener and antacid from particle to particle; drying said free flowing particles; and extruding or compacting said particles into an antacid tablet.

11. The process of Claim 10, wherein mixing of the ingredients and formation of the free flowing particles occurs almost simultaneously.

12. The process of the Claims 10 or 11, wherein the sweetener is selected from the group consisting of glucose, fructose, xylose, ribose, mannose, galactose, invert sugar, sucrose, maltose, lactose, cellobiose, partially hydrolyzed starch, corn syrup and mixtures thereof and the antacid is selected from the group consisting of sodium carbonate, sodium bicarbonate, aluminum hydroxide, magnesium hydroxide, aluminum carbonate, dihydroxyaluminum sodium carbonate, aluminum magnesium hydroxide monohydrate, calcium carbonate, magnesium carbonate, a codried gel of aluminum and magnesium carbonate, and mixtures thereof.

13. The process according to anyone of the Claims 9 to 12, wherein said sweetener is sucrose and said antacid is dihydroxyaluminum sodium carbonate.

14. The process according to anyone of the Claims 9 to 13, wherein a disintegrant is mixed with the ingredients prior to subjecting the ingredients to high shear mixing and said disintegrant is selected from the group consisting of corn starch, potato starch, modified corn starch, bentonite, microcrystallines cellulose, alginates, agar, guar, locust bean, karaya, pectin, tragacanth, and mixtures thereof.

15. The process of Claim 14, wherein said disintegrant is corn starch.

16. A comestible tablet produced by a process as claimed in anyone of the Claims 1 to 15.

17. An antacid tablet produced by the process of Claim 16.

18. A continuous process for producing consumable free flowing particles comprising substantially uniformly distributed ingredients, said process comprises mixing the ingredients together using high intensity mixing or under conditions of high shear, while atomizing a solvent for at least one of the ingredients into the ingredients; and mixing the ingredients until particles are obtained which are free flowing and substantially uniform in the distribution.

19. A process as claimed in Claim 18, wherein ingredients are mixed as described in anyone of the Claims 1 to 8.

0 275 834

European Patent Office

EUROPEAN SEARCH REPORT

Application Number

EP 87 81 0776

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | JOURNAL OF PHARMACEUTICAL SCIENCES, vol. 53, no. 3, March 1964, pages 320-324; A.S. RANKELL et al.: "Continuous production of tablet granulations in a fluidized bed II" * Page 320, column 2; page 322, column 1 * | | A 61 K 9/20<br>A 61 J 3/10 |
| A | DE-A-1 767 301 (GUSTAV SPANGENBERG MASCHINENFABRIK GmbH) | | |
| A | P.H. LIST et al.: "Hagers Handbuch der pharmazeutischen Praxis", 4th edition, vol. 7, part A, 1971, Springer-Verlag, Berlin, DE * Page 714, middle; page 732, line 1 * | | |

TECHNICAL FIELDS SEARCHED (Int. Cl.4)

A 61 K
A 61 J

The present search report has been drawn up for all claims

| Place of search | Date of completion of the search | Examiner |
|---|---|---|
| THE HAGUE | 06-04-1988 | BENZ K.F. |